# EUROPEAN PATENT APPLICATION

(11) **EP 3 970 669 A1**
(43) Date of publication of application: **23.03.2022**
(21) Application number: 20197283.3
(22) Date of filing: 21.09.2020
(51) Int. Cl.: A61F 2/38, A61F 2/30

(54) **STIFLE JOINT STABILIZING MENISCUS PROSTHESIS**

(71) Applicant: Atro Medical B.V., 6534 AT Nijmegen (NL)
(72) Inventor: VAN DER ZANDEN, Antonius Christiaan, 5384 LS Heesch (NL); VAN DER VEEN, Albert, 1181 PB Amstelveen (NL); TRYFONIDOU, Meropi Adriana, 3435 RS Nieuwegein (NL); MEIJ, Björn Petrus, 3722 GM Bilthoven (NL); VAN TIENEN, Tony George, 6542 RJ Nijmegen (NL)
(74) Representative: Nederlandsch Octrooibureau

(57) **Abstract**

A prosthesis for a mammal comprises a prosthesis body (302) with a dorsal surface (311) and a ventral surface (312) shaped to fit in a joint between a tibia and a femur so that the dorsal surface faces a distal end of the femur and the ventral surface faces a proximal end of the tibia. At least one fixation element (303) is provided for fixing the prosthesis body (302) to the tibia (150), wherein the dorsal surface (311) is inclined in a dorsal direction towards a caudal edge of the prosthesis body, to stabilize the femur.

## Description

### FIELD OF THE INVENTION

The invention relates to a prosthesis for mammal. The invention relates in particular to a meniscus prosthesis for a stifle joint or knee joint of a mammal.

### BACKGROUND OF THE INVENTION

In mammals, such as dogs and humans, the stifle joint stability after (partial) rupture of the cranial cruciate ligament is reconstructed by surgical interventions. In dogs these include Tibial Plateau Leveling Osteotomy (TPLO), tibial tuberosity advancement (TTA), Triple Tibial Osteotomy (TTO), and intra-articular repair techniques and extra-articular repair techniques with autograft or synthetic materials. These existing techniques require invasive approaches with sawing bones and metal fixation plates.

There is a need for an improved less invasive treatment of knee problems related to joint instability in mammals.

A meniscus prosthesis is known from EP 3 116 448 B1 in the name of Atro Medical B.V., which discloses a meniscus prosthesis comprising an arc-shaped meniscus prosthesis body having a main portion comprising a reinforcing part and two end portions comprising fixation parts, wherein the main portion comprises a part made of a first biocompatible, non-resorbable material extending between the two end portions, wherein the reinforcing part and the fixation parts are made of a second biocompatible, non-resorbable material and wherein the reinforcing part extends between the fixation parts and wherein the fixation parts have a through hole. However, veterinary medicine presents different challenges than human medicine.

### SUMMARY OF THE INVENTION

It is an object of the present invention to provide an improved prosthesis for a mammal. In order to address this concern, a prosthesis for a mammal is provided, the prosthesis comprising:
a prosthesis body with a dorsal surface and a ventral surface shaped to fit in a joint between a tibia and a femur so that the dorsal surface faces a distal end of the femur and the ventral surface faces a proximal end of the tibia; and
at least one fixation element (303) for fixing the prosthesis body to the tibia,
wherein the dorsal surface is inclined in a dorsal direction towards a caudal edge of the prosthesis body, to stabilize the stifle joint.

The inclination towards the caudal edge provides stabilization of the stifle joint, because it prevents the distal end of the femur from dislocating in caudal direction with respect to the proximal end of the tibia. The fixation element ensures that a force applied on the inclined caudal edge does not cause the prosthesis to slide over the tibia, thus further stabilizing the joint. Implantation of the prosthesis set forth may provide joint stability, may prevent progression of osteoarthritis, and/or may prevent secondary meniscus lesions and associated secondary interventions.

The dorsal surface may be inclined in the dorsal direction also towards a medial edge of the prosthesis body and/or towards a cranial edge of the prosthesis body. This helps to further stabilize the joint in the medial and/or cranial directions, respectively.

The dorsal surface may protrude in the dorsal direction more at the caudal edge of the prosthesis body than at the medial edge of the prosthesis body and the cranial edge of the prosthesis body. This is because the force exerted by the femur on the prosthesis may be largest in caudal direction. The more protrusion at the caudal edge helps to protect against in-plane movement of the femur with respect to the tibial plateau.

The prosthesis body may have a wedge shape wherein a thickness of the prosthesis body increases from a center of the prosthesis body towards the caudal edge, medial edge, and cranial edge. This may further reinforce the prosthesis body. In certain embodiments the prosthesis body can rest on the surface of the tibial plateau.

For example, the prosthesis is a medial meniscus prosthesis for a stifle joint. Stifle joints of quadrupeds may be vulnerable to instability, due to for example their body conformation, especially after (partial) cranial cruciate ligament rupture. The prosthesis may provide stability to the joint. Further, in certain embodiments, the prosthesis may halt or reduce the development of osteoarthritis and, optionally, related chronic lameness.

At least one of the at least one fixation elements may be located at a cranial end of the prosthesis body or at a cranial horn position of a corresponding native meniscus. These are examples of locations for a fixation element that are particularly well-suited to provide stability to the prosthesis and joint.

The at least one fixation element may comprise a through hole in the prosthesis body. This allows to fix the prosthesis body by means of an engaging element. For example, the fixation element may further comprise a screw, pin, cord or nail, to fit through the through hole.

The ventral surface of the prosthesis may be shaped to match a shape of a tibial plateau. This provides for a good fit of the prosthesis, directly on top of the tibial plateau, for example as a replacement of a removed meniscus.

Alternatively, the ventral surface may be shaped to match a shape of a native meniscus on top of a tibial plateau. This provides for a good fit of the prosthesis, on top of the meniscus. This allows the prosthesis to be used, for example, to stabilize the joint after (partial) cruciate ligament rupture, if the meniscus is still relatively healthy. For example, the prosthesis may be implanted on top of a native meniscus, which may be intact or slightly injured, for example.

As mentioned before, the prosthesis may be configured to replace a stabilizing function of cruciate ligaments of the mammal. However, this is not a limitation. The prosthesis may also be used, for example, to enhance the stabilizing function of cruciate ligaments or to stabilize the joint in general.

The dorsal surface may comprise a rim at the caudal edge of the prosthesis body, wherein the rim is shaped to fit around and extend beyond at least part of an edge of the distal end of the femur, to provide a counter pressure to the distal end of the femur to protect the distal end of the femur from displacement in caudal direction.

According to another aspect of the invention provides the prosthesis set forth for use in a method of treating osteoarthritis. For example, by implanting the prosthesis, development of osteoarthritis may be halted or slowed down.

The person skilled in the art will understand that the features described above may be combined in any way deemed useful. Moreover, modifications and variations described in respect of the system may likewise be applied to the method and to the computer program product, and modifications and variations described in respect of the method may likewise be applied to the system and to the computer program product.

### BRIEF DESCRIPTION OF THE DRAWINGS

In the following, aspects of the invention will be elucidated by means of examples, with reference to the drawings. The drawings are diagrammatic and may not be drawn to scale. Throughout the drawings, similar items may be marked with the same reference numerals.
Fig. 1 shows a dorsal view of menisci and ligaments of the left stifle joint on the proximal end of the tibia.
Fig. 2 shows a medial perspective view of the left stifle joint of a dog.
Fig. 3 shows a first example of a meniscus prosthesis.
Fig. 4A shows a medial view of the stifle joint with the first example of the meniscus prosthesis.
Fig. 4B shows the meniscus prosthesis of the first example in greater detail.
Fig. 4C shows the meniscus prosthesis of the first example superposed on the native meniscus.
Fig. 5 shows a second example of a meniscus prosthesis.
Fig. 6 shows a third example of a meniscus prosthesis.
Fig. 7 shows a medial cross-sectional view of the left stifle joint of a dog with the meniscus prosthesis, according to the third example.
Fig. 8 shows a medial cross-sectional view of the left stifle joint of a dog with a meniscus prosthesis, according to a fourth example.
Fig. 9 shows a sketch of a cross section of the third example of the meniscus prosthesis in sagittal plane.
Fig. 10A shows a frontal view of a quadruped.
Fig. 10B shows a side view of the quadruped.
Fig. 10C shows a frontal view of a human.
Fig. 10D shows a side view of a human.

### DETAILED DESCRIPTION OF EMBODIMENTS

Certain exemplary embodiments will be described in greater detail, with reference to the accompanying drawings. The matters disclosed in the description, such as detailed construction and elements, are provided to assist in a comprehensive understanding of the exemplary embodiments. Accordingly, it is apparent that the exemplary embodiments can be carried out without those specifically defined matters. also, well-known operations or structures are not described in detail, since they would obscure the description with unnecessary detail.

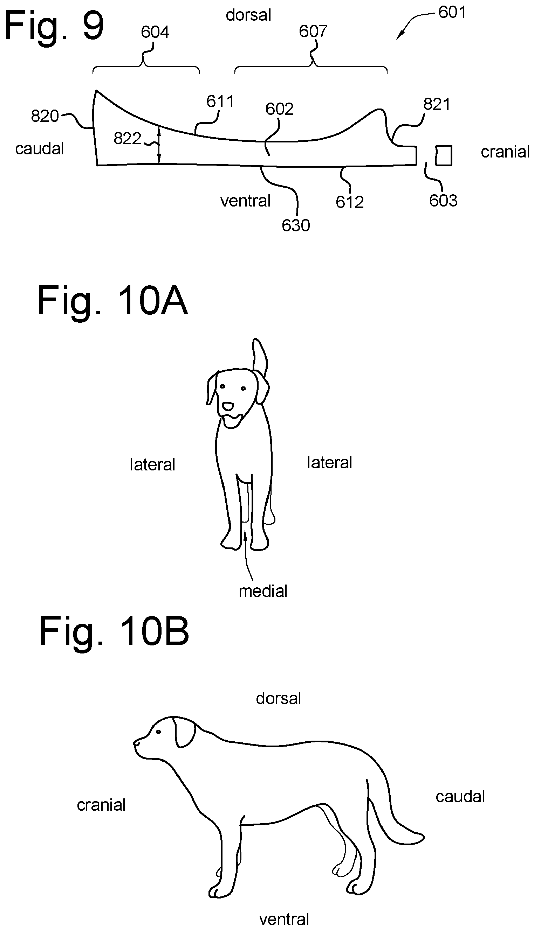

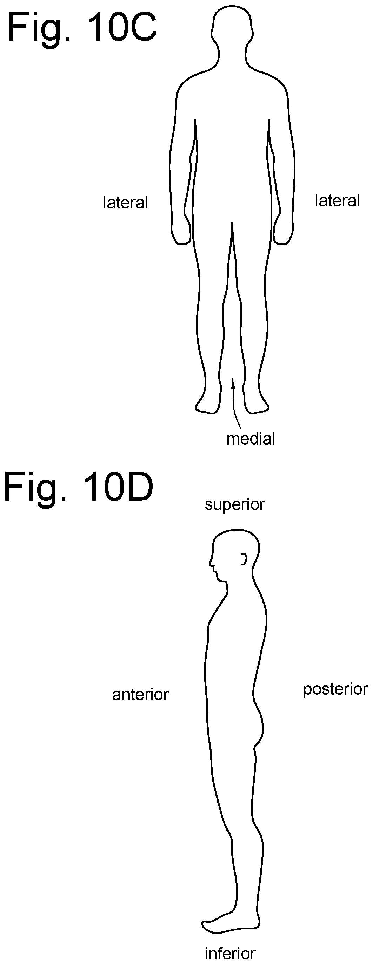

Fig. 10 illustrates names of orientations in mammals used throughout this document. Fig. 10A shows a frontal view of a quadruped, such as a dog. Fig. 10B shows a side view of the quadruped. Fig. 10C shows a frontal view of a human. Fig. 10D shows a side view of a human. Regarding the quadruped, orientations include dorsal and ventral to denote top and bottom side, respectively. Cranial and caudal indicate front and hind sides, respectively, as indicated in Fig. 10B. Lateral indicates the sides of the quadruped, and medial indicates more towards the inside, as indicated in Fig. 10A.

Regarding humans, orientations include superior and inferior to indicate top and bottom, respectively. Expressions anterior and posterior may be used to denote front and back sides, respectively, as illustrated in Fig. 10D. Lateral indicates the sides of the human, and medial indicates more towards the inside, as indicated in Fig. 10C.

Throughout this disclosure, the terminology for quadrupeds is used. However, the same technology may be applied on prostheses for humans. In that case, the expression 'dorsal' may be replaced by 'superior'; the expression 'caudal' may be replaced by 'posterior'; the expression 'ventral' may be replaced by 'inferior', and the expression 'cranial' may be replaced by 'anterior'. The expressions 'lateral' and 'medial' are used in the same way for quadrupeds and humans.

Certain embodiments of the present disclosure relate to the design of an implant which can stabilize and decrease pain in the stifle joint. Additionally or alternatively, the implant may help to halt or slow down the development of osteoarthritis secondary to joint instability and misfit of the joint structures. The stifle joint of certain animals, such as dogs and horses, corresponds to the knee joint in humans. The implant may combine the biomechanical functions of the Cranial Cruciate Ligament (CCL) and the medial meniscus. To that end, the implant may support rotational and translational stability of the femur with respect to the tibia. Further, the implant may distribute the loads imposed on the tibia by the femur in case of joint instability. Such joint instability may be due, for example, to an absent or malfunctioning native meniscus and/or CCL. The implant may comprise a caudal rim, which prevents the proximal tibia from shifting cranially with respect to the femur. This stabilizing function may be provided by the implant's asymmetric geometry. Moreover, the stiffness of the material of the implant can be used to further improve the stability. For example, by choosing a relatively stiff material for (certain regions of) the implant (e.g. stiffer than native meniscus tissue), in combination with the rim, shifting of the tibia with respect to the femur at the stifle joint may be reduced or eliminated. The implant may be fixed on the tibia on at least one location, preferably on the cranial (or anterior) edge of the tibia.

In certain embodiments, a prosthesis for a stifle joint of a mammal comprises a prosthesis body with two surfaces. The prosthesis has a complex shape with a dorsal surface and a ventral surface. When implanted in a stifle joint or knee joint, the dorsal surface may contact the femur, and the ventral surface may contact the tibial plateau. The prosthesis may be fixedly positioned by fixation element(s). The fixation elements may fix the prosthesis body to a bone adjacent to the ventral surface, in particular the tibia. For example, the prosthesis may be placed in a medial compartment of the stifle joint. For example, the prosthesis may be placed in the place of the medial meniscus or in addition to the medial meniscus.

From the lateral side towards the periphery of the prosthesis, which corresponds with the medial, cranial and caudal sides of the joint, the height of the prosthesis in dorsal direction is gradually increasing. With this increment in height, or inclination of the dorsal surface of the prosthesis body in dorsal direction, a "grip portion" is created that provides grip to the adjacent bones, in particular the femur, and has the ability to restrain movement of femur and tibia within a physiologic range of motion of the joint. The combination of the improved grip thanks to the inclination and the fixation element(s) may lead to more stability in the stifle joint while maintaining a physiologic range of motion. The inclination of the dorsal surface of the prosthesis body, together with the fixation element(s), may protect the stifle against large stresses that may occur where the tibia and the femur are joined in the stifle joint. Furthermore, the inclination may prevent the femur from slipping away.

This implant design may be less invasive than existing procedures to stabilize the stifle joint after (partial) cranial cruciate ligament rupture. Moreover, the implant design may provide sufficient stabilization of the stifle joint. TPLO, TTA, and TTO involve bony cuts in the tibia near the stifle joint, or repositioning of the patella ligament attachment on the tibia to adapt the stifle joint biomechanics. In certain embodiments, such procedures may be avoided by the present prosthesis.

The dorsal surface of the prosthesis can have an increment in height in the dorso-ventral direction (i.e. sagittal plane), forming a grip portion of the prosthesis. The grip portion with the increment in height may be formed at least at the caudal end of the prosthesis body. The grip portion may be at the caudal end of the prosthesis body; this does not exclude increments of height in the ventro-dorsal direction at other portions of the periphery of the prosthesis, such as the medial and cranial ends of the prosthesis. The increment in height may be larger at the caudal end of the prosthesis than at the medial and cranial ends of the prosthesis, restricting the shift of the femur backwards, the common instability after CCL lesions. In this way, the femur may be supported by the grip portion and restrained in its movement within the physiologic and healthy range of motion.

In certain embodiments, the implant may comprise a C-shape, resembling the medial meniscus, with the inner edge being the lateral edge and the outer edge comprising the caudal, medial, and cranial edges. The height of the dorsal surface may be smaller at the inner edge of the C-shape (lateral edge) than at the outer edge of the C-shape (the caudal, medial, and cranial edges).

In certain embodiments, the outer edges extend further in the dorsoventral direction from the implant body than the inner part of the prosthesis. Moreover, the caudal edge of the dorsal surface may extend even further in the dorsoventral direction from the implant body than the cranial and medial edge. This way, the grip on the bones is largest in the direction where the largest shear stresses may occur. For example, this may prevent the medial femoral condyle to slide backwards on the tibia. This may occur after cranial cruciate ligament rupture in, for example, dogs and other quadrupeds, and causes instability of the joint. In humans, this problem may occur for example due to a rupture of the anterior cruciate ligament.

The grip portion may have a wedge shape. This way, by gradually incrementing in height further towards the cranial, lateral and caudal edge, sharp edges on the caudal surface may be avoided.

The fixation element may be located at a cranial end of the prosthesis body. When the maximum height of the grip portion is located at the caudal end, the forces exerted on the caudal end by the femur and on the cranial end by the tibia via the fixation element may keep the implant firmly in the correct place. However, the fixation element may be located anywhere on the prosthesis or prosthesis body. Moreover, the presence of one or more extra fixation elements is not excluded.

The thickness of the prosthesis body, measured as a distance between the dorsal surface and the ventral surface, may be greater at the edge of the prosthesis body, in particular the caudal, medial, and cranial edges (with the most pronounced increment in the caudal edge) than in the remainder of the prosthesis body.

The ventral surface of the prosthesis may be shaped to match a shape of the tibial plateau. This improves the fit of the prosthesis, in case for example the live meniscus is (at least partially) removed.

The ventral surface may be shaped to match a shape of a meniscus on top of the tibial plateau. This improves the fit of the prosthesis, when the prosthesis is implanted on top of the resident meniscus.

The dorsal surface may be shaped to match a shape of a distal end of the femur. The grip portion may comprise a rim, shaped to fit around at least part of an edge of the distal end of the femur, to provide a counter pressure to the distal end of the femur to protect it against shear stress with respect to the proximal end of the tibia.

The prosthesis may be configured to replace the function of cruciate ligaments of the mammal: protection against shear stress is primarily a task of the cruciate ligaments. The presented prosthesis may be used to provide that protection when the natural cruciate ligaments are damaged (e.g. partial or complete rupture). Certain embodiments comprise a polymer implant body with a concave-shaped upper surface on which the distal end of the femur is positioned and a bottom surface contacting the tibial plateau. The implant may be higher at the circumference than in the center and higher at the caudal side than the cranial side. The sagittal cross-section of the implant may be wedge-shaped from center to the peripheral rim. The meniscus implant may maintain its position on the tibial plateau by a fixation device.

In certain embodiments, the prosthesis body may be arc-shaped, e.g. resembling the shape of the meniscus. In that case, the center 230 of the prosthesis body may be at or near the lateral edge of the prosthesis body..

The edges of the anatomically shaped implant may provide a restriction to the spherical medial compartment of the distal femur. The wedge-shaped caudal edge of the implant levels the tibial plateau and limits the caudal drift of the femoral compartment. The implant may be fixed to the tibia in order to further prevent the femur from sliding down the tibia and/or dislocating from it. As such, the implant may replace the cranial cruciate ligament.

In certain embodiments, the anatomically shaped dorsal surface of the prosthesis body surrounds the medial stifle compartment of the distal femur and distributes contact stresses over the femoral and underlying tibia cartilage.

In certain embodiments, the anatomically shaped ventral surface of the implant adapts to the medial proximal tibia and distributes the contact stresses over the tibia cartilage.

The fixation device keeps the implant on its position on the tibia and consequently retains the cranial position of the femur on the tibia. This prevents translations that would otherwise lead to high shear stresses on cartilage of the stifle joint generating osteoarthritis or lead to frequent giving way, i.e. dislocation of the femur with respect to the tibia.

In certain embodiments, the flexibility of the implant provides conformity to the femoral and tibia cartilage surfaces in the stifle joint and, as such, transfer of forces from femur to the tibia. For example, the flexible implant may comprise a polymer.

The implant material properties may be chosen so that the implant material limits the movement of the femoral condyle.

A caudal, wedge-shaped cross-section of the implant, together with the optional fixation to the tibial plateau, provides sufficient limitation in freedom of movement of the femur with respect to the tibia. This creates a biomechanically stable stifle joint, even if the cranial cruciate ligament is absent (e.g. due to damage).

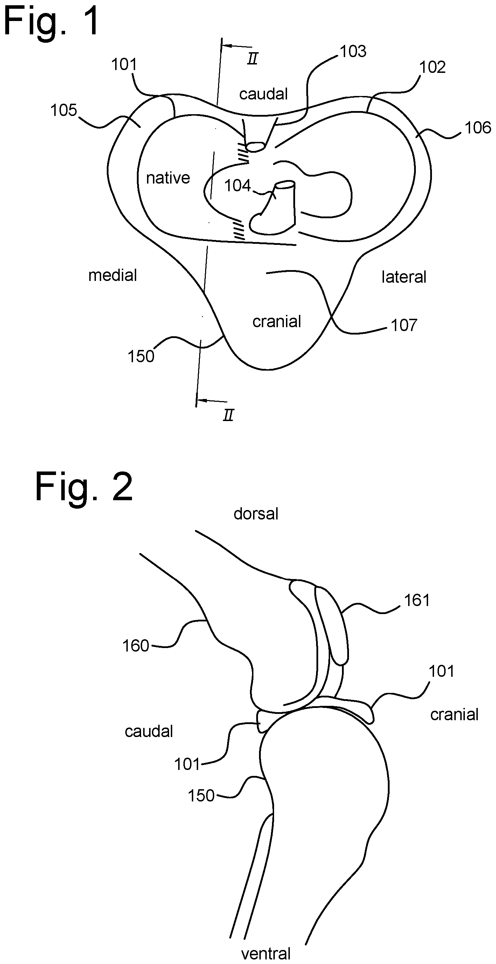

Fig. 1 shows a dorsal view of menisci and ligaments of the left stifle joint on the proximal end of the tibia 150. In particular, shown are the medial meniscus 101, which rests on the medial condyle 105 of the tibia, the lateral meniscus 102, which rests on the lateral tibial plateau 106, a part of the cranial cruciate ligament 103, a part of the caudal cruciate ligament 104, and the cranial intercondylar area 107. Throughout this disclosure, the drawings show the left stifle joint. The same or similar techniques may be applied to the right stifle joint or to a left or right knee.

Fig. 2 shows a medial view of the left stifle joint of a dog. The figure shows a proximal part of the tibia 150, a distal part of the femur 160, the patella 161, and the medial meniscus 101.

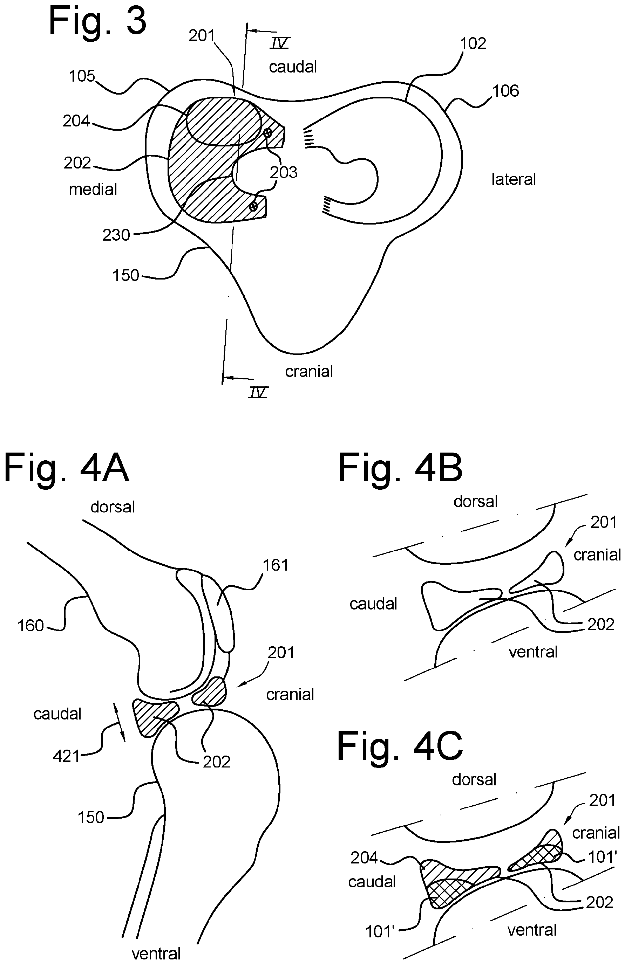

Fig. 3 shows again a dorsal view of the stifle joint on the proximal end of the tibia 150. However, the medial meniscus 101 of the medial compartment of the left stifle joint is replaced by a first example of a meniscus prosthesis 201. The implant 201 comprises an implant body 202 and fixation means 203. The two fixation means 203 keep the implant body 202 attached to the tibia 150.

Fig. 4A shows a medial view of the stifle joint with the first example of the meniscus prosthesis 201. The fixation means 203 are not visible in this cross section. As shown in Fig. 4, the width 421 of the prosthesis 201 increases from the center towards the edge. The greatest width 421 is greatest at the caudal end of the meniscus prosthesis body 202. The dorsal surface of the prosthesis body is inclined in the dorsal direction towards the caudal edge of the prosthesis body 202. The dorsal surface of the prosthesis body extends upwards (in dorsal direction) near the caudal end of the prosthesis body. The dorsal surface may thus form a rim that fits around the distal end of the femur.

Fig. 4B shows the meniscus prosthesis 202 in greater detail.

Fig. 4C shows the meniscus prosthesis 201 superposed with the anatomical meniscus 101', for purpose of illustration only. The meniscus 101' is actually replaced by the meniscus prosthesis 202. However, the drawing shows that the grip portion 204 of the meniscus prosthesis 202 has a greater height than the anatomical meniscus 101', for improved grip of the femur with respect to the tibia.

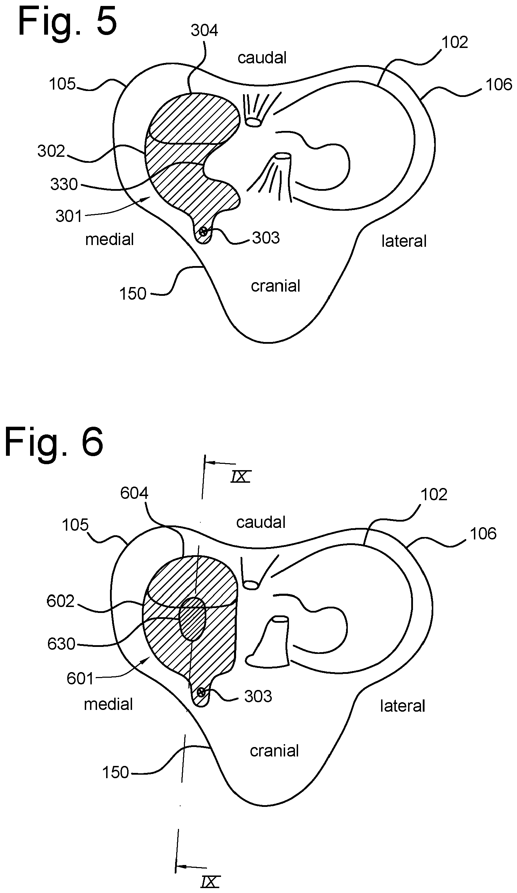

Fig. 5 shows again a dorsal view of the stifle joint on the proximal end of the tibia 150. However, the medial meniscus 101 of the medial compartment of the left stifle joint is replaced by a second example of a meniscus prosthesis 301. The meniscus prosthesis 301 comprises an implant body 302 and fixation means 303 near a cranial edge of the implant body 302. A grip portion 304 of the prosthesis body has a greater thickness and extends in dorsal direction further in comparison to the remaining portion outside the grip portion 304. The thickness of the grip portion 304 increases towards the caudal end of the prosthesis body 302. The general shape of the prosthesis body in dorsal cross section corresponds to that of an anatomical meniscus. The fixation means 303 protrudes from the anatomically shaped prosthesis body in cranial direction.

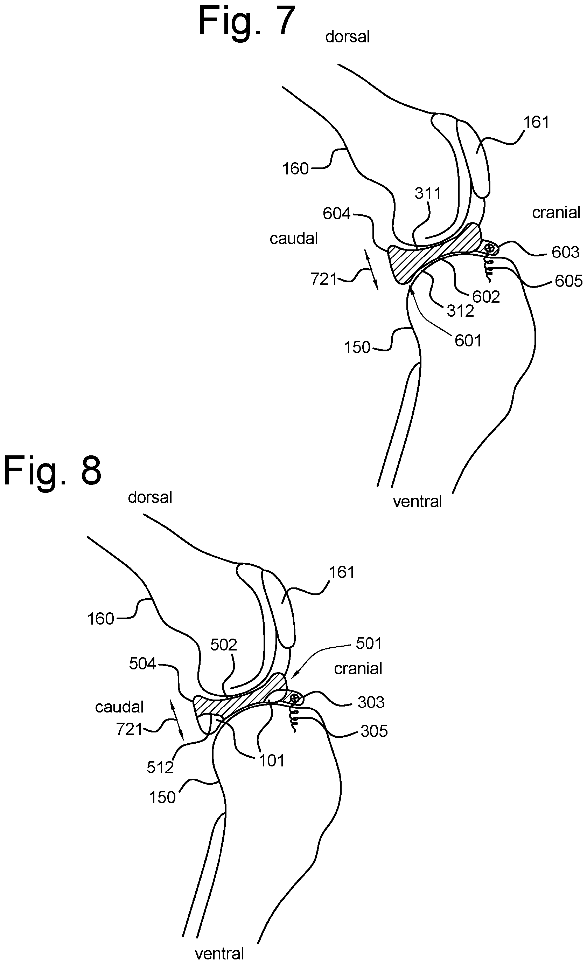

Fig. 6 shows again a dorsal view of the stifle joint on the proximal end of the tibia. However, in Fig. 6, the medial meniscus 101 of the medial compartment of the left stifle joint is replaced by a third example of a meniscus prosthesis 601. The prostheses bodies 201 and 301 of the first and second example are anatomically shaped, with an arc shape or crescent shape. However, the prosthesis body 602 of the third example is discoid in shape. The thickness of the prosthesis body in the center 630 of the prosthesis body is smaller than at the circumference of the prosthesis body. The thickness in the grip portion 604 increases gradually up to the posterior edge of the prosthesis body, where the thickness is greatest. That is, the dorsal surface is inclined in dorsal direction towards the edge of the prosthesis body, in particular towards the caudal edge. Fig. 7 shows a medial view of the left stifle joint of a dog with the meniscus prosthesis 601, according to the third example. The prosthesis 601 has a higher caudal edge 604 of the implant with a greater thickness 721, to resist caudal translation of the femur 160 with respect to the tibia 150. The fixation means 603 near the cranial end of the implant 602 comprises a screw 605 which extends through an opening (e.g. through hole) of the implant 602 into the proximal end of the tibia 150.

Fig. 8 shows a medial view of the left stifle joint of a dog with a meniscus prosthesis 501, according to a fourth example. The top view of this meniscus prosthesis 501 would be similar to the discoid prosthesis 301 of the third example, shown in Fig. 5. In yet another embodiment, the top view could be similar to one of the arc shaped implant bodies. Also the shape of the dorsal surface of the prosthesis body according to the fourth example can be similar to the dorsal surface of the prosthesis body in one of the other examples. In this fourth example, the native meniscus 101 is still in place, and a prosthesis body 502 has been placed on top of the native meniscus 101. Again shown are the exemplary fixation means 303 with screw 305. On the caudal side, it is visible that the dorsal surface, facing the distal end of the femur 160, has a higher dorsal offset 504 than the native meniscus. The dorsal surface of the prosthesis body 502 is inclined in dorsal direction towards the caudal edge of the prosthesis body. It is observed that other embodiments, such as the first and second example, may be modified to be adapted to be used on top of the anatomical meniscus, like the fourth example. In particular, the ventral surface (512) of the prosthesis body may be shaped to match the shape of the dorsal surface of the native meniscus (101) and the underlying tibial plateau.

Fig. 9 shows a sketch (not drawn to scale) of a possible cross section of the third example of the meniscus prosthesis 601 in sagittal plane. The plane of intersection is indicated in Fig. 6. The implant body has a dorsal surface 611 and a ventral surface 612. The implant body 602 has a greater thickness 822 at the edges 820, 821, compared to the center 630 of the implant body 602. The caudal edge 820 is higher than the remaining edges such as the cranial edge 821. The grip portion 604 at the caudal edge 820 has a wedge shape. The dorsal surface 611 is inclined in a dorsal direction towards the caudal edge 820, in particular in the region 604. The caudal edge 820 extends in a dorsal direction further than the remainder 821, 607 of the implant body 302.

It will be understood that the location(s) of the fixation means, the number of fixation positions, and the method of fixation (e.g. screw, pin, adhesive, or otherwise) can be changed without changing the essence of the present disclosure. This applies to all the embodiments.

Further, it is observed that the fixation means may be included in the caudal part of the implant, e.g. as a peg that can be inserted in the tibia or an attachment point for a bone anchor or suture.

Reinforcement of the wedge-shaped caudal end of the implant may be provided in certain embodiments to better withstand the high shear forces between the distal end of the femur and the proximal end of the tibia. This reinforcement may be realized by using a different material or a higher density material near the wedge-shaped caudal end. The properties of the remaining portion of the implant may be selected, for example, to mimic the strength and stiffness of a native meniscus. In alternative embodiments, all of the implant body 302 may have the same strength, for example similar to the natural meniscus. However, it is also possible to use a different strength, for example stronger than a natural meniscus.

In certain embodiments, the shape of the implant does not follow the anatomical shape of the bones in detail. For example, the implant may be made of a geometrical ring-shape or horse-shoe shape. The wedge and fixation means may still be provided to withstand the shear forces between the distal end of the femur and the proximal end of the tibia.

Fig. 10 illustrates the relation of nomenclature of orientations between mammals on two legs and quadrupeds. For the purpose of consistency of terminology, the quadruped nomenclature has been used throughout the present disclosure. However, this does not mean a limitation. The techniques disclosed herein may also be applied to mammals on two legs, such as humans.

It will be understood that, although the above examples are based on the stifle joint of a dog, a similar implant may be made for other mammals, in particular quadrupeds, such as horses and cats.

The material of the implant may allow sterilization. The material of the implant may be biocompatible. The material of the implant may be nontoxic and bio-inert. Suitable materials for the implant body include hydrogels, thermoplastic materials, polyurethanes, for example.

The height of a meniscus of an average dog (e.g. an average-weight Labrador) may be, for example, 4 mm. For example, for such a dog, an appropriate height of the grip portion of the meniscus prosthesis may be 5 mm or greater. This dimension is purely given by means of example only.

The examples and embodiments described herein serve to illustrate rather than limit the invention. The person skilled in the art will be able to design alternative embodiments without departing from the spirit and scope of the present disclosure, as defined by the appended claims and their equivalents. Reference signs placed in parentheses in the claims shall not be interpreted to limit the scope of the claims. Items described as separate entities in the claims or the description may be implemented as a single hardware or software item combining the features of the items described.

## Claims

1. A prosthesis for a mammal, comprising
a prosthesis body (302) with a dorsal surface (311) and a ventral surface (312) shaped to fit in a joint between a tibia and a femur so that the dorsal surface faces a distal end of the femur and the ventral surface faces a proximal end of the tibia; and
at least one fixation element (303) for fixing the prosthesis body (302) to the tibia (150),
wherein the dorsal surface (311) is inclined in a dorsal direction towards a caudal edge of the prosthesis body, to stabilize the stifle joint.

2. The prosthesis of claim 1, wherein the dorsal surface (311) is inclined in the dorsal direction also towards a medial edge of the prosthesis body or towards a cranial edge of the prosthesis body.

3. The prosthesis of claim 2, wherein the dorsal surface (311) protrudes in the dorsal direction more at the caudal edge of the prosthesis body than at the medial edge of the prosthesis body and the cranial edge of the prosthesis body.

4. The prosthesis of any preceding claim, wherein the prosthesis body has a wedge shape wherein a thickness of the prosthesis body increases from a center (330) of the prosthesis body (302) towards the caudal edge, medial edge, and cranial edge.

5. The prosthesis of any preceding claim, wherein the prosthesis is a medial meniscus prosthesis for a stifle joint.

6. The prosthesis of any preceding claim, wherein at least one of the at least one fixation element (303) is located at a cranial end of the prosthesis body (302) or at a cranial horn position of a corresponding native meniscus.

7. The prosthesis of any preceding claim, wherein the at least one fixation element (303) comprises a through hole in the prosthesis body (302).

8. The prosthesis of claim 7, wherein the fixation element (303) further comprises a screw (305), pin, cord, or nail, to fit through the through hole.

9. The prosthesis of any preceding claim, wherein the ventral surface is shaped to match a shape of a tibial plateau.

10. The prosthesis of any one of claims 1 to 8, wherein the ventral surface (512) is shaped to match a shape of a meniscus (101) on top of a tibia (150) plateau.

11. The prosthesis of any preceding claim, wherein the prosthesis is configured to replace a stabilizing function of cruciate ligaments of the mammal.

12. The prosthesis of any preceding claim, wherein the dorsal surface (311) comprises a rim at the caudal edge of the prosthesis body, wherein the rim is shaped to fit around and extend beyond at least part of an edge of the distal end of the femur, to provide a counter pressure to the distal end of the femur to protect the distal end of the femur from displacement in caudal direction.

13. The prosthesis of any preceding claim for use in a method of treating osteoarthritis.
